Europäisches Patentamt

European Patent Office

Office européen des brevets

(19)

(11) Veröffentlichungsnummer: **0 180 743 B1**

(12) **EUROPÄISCHE PATENTSCHRIFT**

(45) Veröffentlichungstag der Patentschrift: **06.11.91**

(21) Anmeldenummer: **85111734.1**

(22) Anmeldetag: **17.09.85**

(51) Int. Cl.5: **C12N 1/04, C12N 11/04,**
**A61K 9/50, A61K 35/74,**
**A01N 63/00, A23K 1/00**

Die Akte enthält technische Angaben, die nach
dem Eingang der Anmeldung eingereicht wurden
und die nicht in dieser Patentschrift enthalten sind.

(54) **Lebende Mikroorganismen enthaltendes flüssiges Präparat, Verfahren zu dessen Herstellung, lebende Mikroorganismen enthaltendes pelletiertes Produkt und Verfahren zu dessen Herstellung.**

(30) Priorität: **21.09.84 CH 4545/84**

(43) Veröffentlichungstag der Anmeldung:
**14.05.86 Patentblatt 86/20**

(45) Bekanntmachung des Hinweises auf die
Patenterteilung:
**06.11.91 Patentblatt 91/45**

(84) Benannte Vertragsstaaten:
**AT BE CH DE FR GB IT LI LU NL SE**

(56) Entgegenhaltungen:
**EP-A- 0 006 671**      **EP-A- 0 065 376**
**EP-A- 0 097 571**      **WO-A-83/03102**
**CH-A- 457 332**        **GB-A- 2 016 043**
**LU-A- 60 508**

(73) Patentinhaber: **Cernitin S.A.**

**CH-6911 Barbengo(CH)**

(72) Erfinder: **Lewenstein, Ari**
**Via Marco da Carona 5**
**CH-Lugano(CH)**

(74) Vertreter: **Blum, Rudolf Emil Ernst et al**
**c/o E. Blum & Co Patentanwälte Vorderberg**
**11**
**CH-8044 Zürich(CH)**

## Beschreibung

### Hintergrund der Erfindung

Die vorliegende Erfindung betrifft ein lebende Mikroorganismen enthaltendes Präparat, das bei Zimmertemperatur lagerfähig ist, in flüssiger Form vorliegt und dadurch bei seiner Verwendung leicht dosierbar ist. Die lebenden Mikroorganismen dieser Präparate sind von einer wasserlöslichen oder in Wasser quellenden Schutzschicht umhüllt und in einer flüssigen Oelphase suspendiert, in welcher die Schutzschicht unlöslich ist.

Des weiteren betrifft die Erfindung ein Verfahren zur Herstellung des lebende Mikroorganismen enthaltenden Präparates und ferner ein pelletiertes diätetisches Nahrungsmittel oder einen Futtermittelzusatz, der die üblichen proteinhaltigen Komponenten, stärkehaltigen Komponenten, sowie gegebenenfalls Mineralstoffe und Vitamine enthält und ausserdem lebende Mikroorganismen, die von der wasserlöslichen oder in Wasser quellenden Schutzschicht, die in Oel unlöslich ist, umhüllt sind. Ausserdem betrifft die Erfindung ein Verfahren zur Herstellung derartiger pelletierter diätetischer Nahrungsmittel.

Es ist bekannt, dass Mikroorganismen, beispielsweise Bakterien, bei sehr tiefen Temperaturen, die weit unter dem Gefrierpunkt liegen, einige Zeit hindurch gelagert werden können und dennoch ihre Lebensfähigkeit behalten. Bei der praktischen Verwendung von solchen lebende Mikroorganismen enthaltenden Präparaten ist es jedoch nachteilig, dass eine Lagerung im tiefgefrorenen Zustand nötig ist.

Es ist ferner bekannt, Mikroorganismen durch Trocknung, insbesondere Gefriertrocknung, für einige Zeit in lebendem Zustand haltbar zu machen. Ein Nachteil dieser durch Gefriertrocknung hergestellten Präparate besteht darin, dass grosse Anteile der lebensfähigen Mikroorganismen dennoch bei einer Lagerung bei Zimmertemperatur ihre Lebensfähigkeit verlieren. Die Hersteller derartiger Präparate verlangen deshalb, dass die Produkte im Kühlschrank aufbewahrt werden, um eine Haltbarkeit während einiger Monate zu gewährleisten. Wenn derartige Präparate in der Landwirtschaft eingesetzt werden, um Nutzpflanzen oder Zierpflanzen gegen den Angriff von Schädlingen zu schützen oder in der Tierhaltung eingesetzt werden, um die Darmflora von Zuchttieren günstig zu beeinflussen, dann stehen häufig keine ausreichenden Kühlkapazitäten zur Verfügung und die Präparate .werden oft von wenig geschulten Personen verwendet, für die es nicht offensichtlich ist, dass die fraglichen Präparate bei Aufbewahrung ausserhalb des Kühlschrankes rasch ihre Aktivität verlieren. Durch die Verwendung von Präparaten, die vorzeitig ihre Aktivität verloren haben und dementsprechend nicht mehr die vermutete Wirksamkeit besitzen, sind im Ackerbau und in der Tierzucht schon grosse Schäden entstanden.

In trockener Form vorliegende Mikroorganismenpräparate, die zur Regulierung der Darmflora dem Tierfutter zugesetzt werden, müssen vor ihrer Verwendung mit entsprechenden Mengen an Wasser vermischt werden. Dabei ergeben sich natürlich häufig Schwierigkeiten durch falsche Dosierung. Ein weiterer Nachteil besteht darin, dass die Gefässe, in denen das Präparat mit Wasser vermischt wurde, nach der Verwendung sorgsam gereinigt werden müssen, um die Mikroorganismen zu entfernen, die an den Gefässwänden haften geblieben sind. Auch eine Verunreinigung der Umgebung, beispielsweise des Stalles, durch Verschütten des wässrigen Mediums, welches die Mikroorganismenpräparate darin dispergiert enthält, muss sorgsam vermieden werden. Dies ist insbesondere dann wichtig, wenn der Mikroorganismus des Präparates ein Bakterium der Klasse Streptokokkus faecium ist, weil Oberflächen, die mit diesem Mikroorganismus verseucht sind, in kurzer Zeit einen äusserst unangenehmen Geruch entwickeln.

Es ist ferner bekannt, dass Mikroorganismen, die in wässrigen Flüssigkeiten dispergiert sind, innerhalb von wenigen Tagen ihre Aktivität verlieren, wenn sie beispielsweise bei Temperaturen im Bereich von 10 - 30° C aufbewahrt werden. Bei diesen Temperaturen führt die Stoffwechselaktivität der Mikroorganismen rasch zu einer Abtötung und Zerstörung der fraglichen Mikroorganismen.

Es ist ferner bekannt, dass lebende Mikroorganismen, die in einer Oelphase aus einem pflanzlichen Oel oder einem Mineralöl dispergiert sind, während gewisser Zeiträume ihre Lebensfähigkeit aufrecht erhalten. Derartige Präparate besitzen gegenüber in trockener Form vorliegenden Mikroorganismenpräparaten den Vorteil einer leichteren Dosierbarkeit und in der Tierzucht ist es nicht nötig, die Präparate mit dem Futtermittel zu vermischen, sondern sie können auch direkt in flüssiger Form an die Tiere verabreicht werden. Dadurch werden die oben beschriebenen Schwierigkeiten bezüglich der Verunreinigung der Umgebung vermieden. Dennoch ist jedoch die Haltbarkeit derartiger bisher beschriebener Präparate unbefriedigend, insbesondere dann, wenn sie ausserhalb des Kühlschrankes gelagert werden. Ein Ziel der vorliegenden Erfindung war es dementsprechend, ein lebende Mikroorganismen enthaltendes Präparat zu entwickeln, bei dem die Mikroorganismen in einer flüssigen Oelphase suspendiert sind, das jedoch gegenüber bisher bekannten entsprechenden Präparaten eine verbesserte Lagerfähigkeit besitzt.

Futtermittelzusätze, die proteinhaltige Komponenten, stärkehaltige Komponenten, sowie gegebenenfalls Mineralstoffe und/oder Vitamine enthalten, werden häufig in pelletierter Form in den Handel gebracht, weil in dieser Form die Verabreichung besonders vorteilhaft ist. Lebende Mikroorganismen konnten jedoch derartigen pelletierten Nahrungsmitteln nicht einverleibt werden, weil sie den Pelletierungsvorgang, der ein Erhitzen nötig macht, nicht oder nur in geringem Masse überlebten.

Es wurde bereits versucht, Mikroorganismen mit einer Schicht aus wachsartigen Materialien zu überziehen und diese mit der Schutzschicht überzogenen Mikroorganismen, zusammen mit den übrigen Komponenten des Futtermittelzusatzes, zu pelletieren. Bei der praktischen Durchführung dieses Verfahrens waren dann aber in den fertigen Futtermittelpellets noch zu wenige lebende Mikroorganismen enthalten.

Ein weiteres Ziel der vorliegenden Erfindung war es, ein pelletiertes diätetisches Nahrungsmittel oder einen pelletierten Futtermittelzusatz zur Verfügung zu stellen, wobei diese Produkte neben den üblichen Energie liefernden Bestandteilen, den üblichen Zusätzen, wie Mineralstoffen und/oder Vitaminen, noch lebende Mikroorganismen enthalten sollen.

Stand der Technik

In der Schweizer Patentschrift Nr. 457 332 wird ein Verfahren zur Konservierung von lebender Hefe beschrieben, die auf einen Wassergehalt von 5 bis 20 % entwässert ist und in einem wasserfreien Fettmaterial, das gegebenenfalls ein Emulgiermittel enthält, dispergiert ist. Gegebenenfalls enthält das Produkt noch rohe Stärke, deren Körnchen vorzugsweise eine ähnliche Grösse besitzen, wie die Hefezellen. Dieser Patentschrift kann kein Hinweis entnommen werden, dass die entsprechenden entwässerten Hefezellen von irgendeiner wasserlöslichen oder in Wasser quellenden Schutzschicht umhüllt sind und bei dem dort beschriebenen Produkt handelt es sich auch nicht um unumhüllte Hefezellen, die in irgendeinem flüssigen Oel dispergiert sind, sondern in Spalte 4, Zeilen 28 - 31 dieser Veröffentlichung wird darauf hingewiesen, dass sich das entsprechende trockene Produkt trotz seines relativ hohen Fettgehaltes nicht fettig anfühlt.

In der belgischen Patentveröffentlichung Nr. 667 201 wird ein Verfahren zur Herstellung von Zusammensetzungen beschrieben, die Bakterien, Enzyme oder Aehnliches enthalten, wobei die fraglichen Zusammensetzungen gegen Feuchtigkeiten und chemische Substanzen der Umgebung durch eine Umhüllung mit einer flüssigen Schutzschicht geschützt sind, die aus einem Oel, beispielsweise Pflanzenöl oder Paraffinöl, oder Propylenglykol (siehe Seite 2, Zeilen 21 bis 24) bestehen kann.

Die französische Patentveröffentlichung Hr. 2 233 941 betrifft Zusammensetzungen, die trockene Hefe und ein geniessbares Fett oder Oel, sowie gegebenenfalls ausserdem Mehl enthalten. Des weiteren können in diesen Zusammensetzungen geringe Mengen an zusätzlichen Bestandteilen enthalten sein, die bei der Verwendung des Produktes die Konsistenz des Teiges günstig beeinflussen, wie zum Beispiel 1-Cystein oder Azodicarbonamid.

In der USA-Patentschrift Nr. 3 034 968 wird ein Verfahren zur Herstellung von lebensfähigen trockenen Mikroorganismen aus der Klasse Bakterien und Schimmelpilze beschrieben, wobei die Trocknung in einem Oel vorgenommen werden kann. Es wurde ferner festgestellt, dass in dem Endprodukt mehr lebende Mikroorganismen enthalten sind, wenn die feuchten Mikroorganismen während des Trockenvorganges mit einem von ihnen abbaubaren Kohlehydrat in Berührung stehen, wie zum Beispiel Saccharose, Dextrose, Maltose und Stärkehydrolysate. Nach dem Trocknungsvorgang werden die Mikroorganismen im allgemeinen von dem Oel befreit, sodass die Endprodukte nurmehr einen Oelgehalt von 10 % oder 5 % oder noch geringere Oelgehalte aufweisen. Früher wurde festgestellt, dass es bei der Gefriertrocknung von verschiedenen feuchten Bakterienstämmen vorteilhaft ist, wenn vorher dem wässrigen Medium verschiedene Materialien zugesetzt werden, wie Glucose, Dextrin, Ammoniumchlorid und Aehnliches (siehe die Veröffentlichungen von Record und Taylor in J. Gen. Microbiol., Band 9, S. 475, 1953 und von Naylor und Smith in J. Bact., Band 52, S. 565, 1946).

In der französischen Patentveröffentlichung Nr. 1 242 371 wird ein Verfahren zur Herstellung einer aktiven Trockenhefe beschrieben, bei dem die Hefe in einer Flüssigkeit, beispielsweise einem essbaren Oel, suspendiert und getrocknet wird. Auch in diesem Fall wurden in dem Endprodukt ein höherer Gehalt an lebenden Hefezellen festgestellt, wenn während des Trocknungsvorganges ein von der Hefe abbaubares Kohlehydrat anwesend war, beispielsweise Zucker oder Maissirup.

Weitere Verfahren, gemäss denen lebende Mikroorganismen haltbar gemacht werden, indem man sie mit einem Oel beschichtet oder in einem Oel suspendiert, sind in der USA-Patentschrift Nr. 1 457 097 und der britischen Patentschrift Nr. 1 151 669 beschrieben.

Die französische Patentveröffentlichung Nr. 2 116 110 betrifft Produkte, bei denen Mikroorganismen in nicht öligen Suspendiermitteln suspendiert sind, wie zum Beispiel in wasserlöslichen Alginaten, Gelatine, natürlichen Gummen, kolloidaler Kieselsäure, Zellulosegummen, sauren Polysacchariden, Polysaccharidderivaten von Algen und insbesondere Natriumalginat. Die Produkte, in denen die Mikroorganismen suspendiert sind, sollen keinerlei Nährwirkung für die Mikroorganismen besitzen (siehe Seite 2, Zeilen 32 bis 36).

Auch in der europäischen Patentveröffentlichung Nr. 0 097 571 werden Mikroorganismenkulturen beschrieben, die in einem Polymergel eingeschlossen sind und deren Wassergehalt unter 0,3 % liegt.

In der PTC-Veröffentlichung Nr. WO 83/03102 wurde ein Verfahren zur Immobilisierung von Biomaterial, wie zum Beispiel tierischen oder pflanzlichen Zellen, Bakterien, Algen, Pilzen, Viren oder Proteinen durch Einkapslung in Polymer-kugeln, bzw. -perlen beschrieben, wobei bei diesem Verfahren das biologische Material zu einer wässrigen Lösung eines Polymers oder von Monomermaterialien zugegeben wird. Man lässt dann das Polymer oder die Monomermaterialien ein Gel bilden und dieses wird denn in einem Dispersionsmedium dispergiert, das in Wasser nicht löslich ist. Als Beispiele für in Wasser nicht lösliche Dispersionsmedien werden gesättigtes oder ungesättigtes Pflanzenöl, tierische Oele oder Fette, Tri-n-butylphosphat, flüssiges Silicon, Paraffinöl oder Phthalsäuredibutylester genannt. In diesem Dispersionsmedium nicht lösliche Polymermaterialien können solche auf Kohlehydratbasis oder Proteinbasis sein, wie zum Beispiel Agar-Agar, Agarose, Carrageen, Chitosan, Gelatine, Kollagen und Fibrinogen.

Der Anspruch 9 dieser Patentveröffentlichung ist auf die nach diesem Verfahren hergestellten immobilisierten Biomaterialien gerichtet, die in einem lebensfähigen Zustand unter Beibehaltung ihrer Vermehrungsfähigkeit in den Polymerkugeln eingekapselt sind und die hergestellt worden sind, indem man sie in einem in Wasser nicht löslichen Medium dispergierte. Aus diesem Anspruch 9 ist nicht ersichtlich, ob das dort erwähnte wasserunlösliche Dispersionsmedium in dem fraglichen immobilisierten Biomaterial noch anwesend ist oder nicht. Aus den Beispielen 1 bis 5 dieser Veröffentlichung ist jedoch ersichtlich, dass die das Biomaterial enthaltenden Polymerperlen relativ gross sind und nach ihrer Dispergierung in der Oelphase aus dieser wieder entfernt werden. Auch bei der in Beispiel 6 dieser Veröffentlichung beschriebenen Ausführungsart werden das Biomaterial enthaltende Gelatinekapseln erzeugt, indem man die Gelatine, welche die lebenden Zellen enthält, in Sojabohnenöl dispergiert, welche als weitere Komponente das Vernetzungsmittel Toluoldiisocyanat zur Vernetzung der Gelatineoberfläche enthält und die erhaltenen Gelatinekapseln werden anschliessend aus der Oelphase entfernt, in Wasser eingebracht und dort erhitzt, um die nicht vernetzte Gelatine in Lösung zu bringen. Auch aus den in den Beispielen 7, 8 und 9 dieser PTC-Veröffentlichung beschriebenen Arbeitsweisen ist klar ersichtlich, dass die gebildeten Polymerkapseln, welche die lebenden Zellen enthalten, aus der zur Dispergierung verwendeten Oelphase wieder entfernt werden.

Dieser Patentveröffentlichung WO 83/03102 kann also kein Hinweis entnommen werden, durch den ein Produkt irgendwie nahegelegt wird, in welchem einzelne lebende Mikroorganismen, die von einer wasserlöslichen oder in Wasser quellenden Schutzschicht umhüllt sind, oder kleine Kügelchen aus derartigen von der Schutzschicht umhüllten Mikroorganismen in einer Oelphase suspendiert sind.

In der europäischen Patentveröffentlichung 0 006 671 ist ein Verfahren zur Konservierung von lebenden Mikroorganismen beschrieben, bei dem eine Suspension der Mikroorganismen zusammen mit einer hochmolekularen quellfähigen wasserlöslichen Trägersubstanz zu einem Granulat getrocknet wird. Als Mikroorganismen sind Hefen und Bakterien bevorzugt und als Trägersubstanz werden vorzugsweise Gelatine, Stärke und/oder Polysaccharide, Pektin und Eiweisslösungen verwendet. Dieser europäischen Patentanmeldung ist jedoch kein Hinweis zu entnehmen, dass das nach dem dort beschriebenen Verfahren erhaltene Granulat in einer Oelphase suspendiert werden soll und ferner ist auch kein Hinweis auf solche Mikroorganismen zu entnehmen, die von einer wasserlöslichen oder in Wasser quellenden Schutzschicht umhüllt sind und in einer Oelphase suspendiert sind.

In der veröffentlichten britischen Patentanmeldung 2 016 043 wird ein Futtermittelzusatz beschrieben, der lebende Bakterien enthält und die Magen-Darm-Flora der Tiere günstig beeinflusst, und dieses Produkt enthält die lebenden Bakterien, eingekapselt in einem Material niedrigen Wassergehalts im Gleichgewicht mit Luft relativ hoher Feuchtigkeit. Das fragliche Einkapslungsmaterial setzt die Bakterien im Verdauungstrakt der Tiere frei.

Als bevorzugte Einkapslungsmittel werden feste Fette, Fettsäuremonoglyzeride, äthoxylierte Fettalkohole, Zucker, sowie feste oder halbfeste Polyäthylenglykole, beispielsweise die Produkte der Markenbezeichnung "Carbowax" genannt. Auf Seite 2, Zeilen 46 bis 48 der genannten britischen Patentveröffentlichung wird darauf hingewiesen, dass zusätzlich zu den Einkapslungsmaterialien noch geringe Mengen an inerten Zusätzen, wie beispielsweise Bindemittel, nützlich sein können und dass auch noch weitere Hilfsmittel beigefügt werden können, wie zum Beispiel pulverförmige Mittel und andere Hilfsmittel.

Der Anspruch 10 der britischen Patentveröffentlichung ist auf entsprechende lebende Bakterien enthaltende Pellets gerichtet und das Pelletierverfahren wird auf Seite 3, Zeile 64, bis Seite 4, Zeile 1, beschrieben. In dieser britischen Patentanmeldung sind jedoch nur Hinweise auf solche pelletierten Produkte enthalten, in welchen die Schutzschicht, welche die Bakterien umhüllt, ein festes oder halbfestes Polyäthylenglykol enthält (siehe die Beispiele 2 und 3 dieser Veröffentlichung). Wie aus der Tabelle 6 auf Seite 5 dieser Veröffentlichung ersichtlich ist, gewährleisten die Polyäthylenoxyd enthaltenden Materialien der Schutzschicht keinen guten Schutz während des Pelletiervorganges, denn ein Produkt, das vor dem Pelletieren noch 200'000 lebende Bakterien pro Gramm enthält, weist unmittelbar nach dem Pelletiervorgang nurmehr 62'000 lebende Bakterien pro Gramm auf, d.h. also zwei Drittel der Bakterien sterben während des Pelletiervorganges ab. Auch das pelletierte Produkt selbst besitzt keine gute Lagerfähigkeit, denn wie aus dieser Tabelle 6 ersichtlich ist, sinkt in dem pelletierten Produkt nach einer Lagerungszeit von nur 5 Wochen der Anteil an lebenden Bakterien noch weiter auf nurmehr 36'000 lebende Bakterien ab, also auf 58 % der lebenden Bakterien, die den Pelletiervorgang noch überlebt hatten.

Bei den erfindungsgemässen pelletierten Produkten wird durch die Verwendung von wasserlöslichen oder wasserquellbaren Schutzschichtmaterialien, die bei Zimmertemperatur fest sind und aus der Gruppe ausgewählt sind, die entsprechende Cellulosederivate, Stärkederivate, Pflanzengummen und Pektine oder Mischungen davon umfasst, erreicht, dass während des Pelletiervorganges nur wenige Mikroorganismen absterben und bei der Lagerung der Pellets der Gehalt an lebenden Mikroorganismen nur geringfügig absinkt.

## Beschreibung der Erfindung

Ziel der vorliegenden Erfindung war es, ein lebende Mikroorganismen enthaltendes Präparat zur Verfügung zu stellen, bei dem die Mikroorganismen in einer flüssigen Oelphase suspendiert sind, wobei dieses Präparat im Vergleich zu bisher bekannten entsprechenden Präparaten, eine wesentlich erhöhte Lagerfähigkeit bei einer Lagerung bei Zimmertemperatur besitzen soll. Ueberraschenderweise zeigte es sich, dass die angestrebten Ziele erreicht werden können, indem man die in trockener Form vorliegenden Mikroorganismen mit mindestens einer wasserlöslichen oder in Wasser quellenden, ebenfalls im wesentlichen trockenen Schutzschicht umhüllt, ehe sie in der flüssigen Oelphase suspendiert werden.

Ein Gegenstand der vorliegenden Erfindung ist daher ein lebende Mikroorganismen enthaltendes Präparat, bei dem die Mikroorganismen in einer flüssigen Oelphase suspendiert sind, das dadurch gekennzeichnet ist, dass es ein bei Zimmertemperatur lagerfähiges Präparat ist, und dass die Mikroorganismen, die in der Oelphase suspendiert sind, in getrockneter Form vorliegen und von mindestens einer wasserlöslichen oder in Wasser quellenden, im wesentlichen wasserfreien Schutzschicht umhüllt sind, die in der Oelphase unlöslich ist, wobei die Oelphase aus einem Mineralöl und/oder pflanzlichen Oel und/oder tierischen Oel, und/oder synthetischen flüssigen Fettsäureester aufgebaut ist, und wobei im trockenen Zustand ein Gewichtsteil an Mikroorganismen von 0,5 bis 10 Gew.-Teilen des Materiales aus der wasserlöslichen oder in Wasser quellenden Schutzschicht umhüllt ist.

Vorzugsweise besteht die Schutzschicht aus dem wasserquellbaren oder wasserlöslichen Material aus einem entsprechenden Produkt, das bei Zimmertemperatur fest ist und das ferner vorzugsweise von den Mikroorganismen nicht abbaubar ist. Bevorzugte Materialien der die Mikroorganismen umhüllenden Schutzschicht sind wasserlösliche Zellulosederivate, wasserlösliche Stärkederivate, wasserlösliche Pflanzengummen, wasserlösliche Pektine oder Mischungen aus zwei oder mehr derartigen wasserlöslichen Prcdukten. Besonders gute Ergebnisse bezüglich der Haltbarkeit der lebende Mikroorganismen enthaltenden Präparate wurden dabei erzielt, wenn als Material für die Schutzschicht wasserlösliche pflanzliche Gummen verwendet werden, insbesondere Gummiarabikum.

Es zeigte sich ferner, dass die Lebensfähigkeit der in den erfindungsgemässen Präparaten enthaltenen Mikroorganismen bei einer Lagerung bei Zimmertemperatur über mehr als zwei Jahre nur dann gewährleistet werden kann, wenn man die von der wasserlöslichen oder wasserquellbaren Schutzschicht umhüllten Mikroorganismen trocknet, beispielsweise gefriertrocknet oder sprühtrocknet und die getrockneten, von der Schutzschicht umhüllten Mikroorganismen dann in der Oelphase suspendiert. In den bevorzugten erfindungsgemässen Präparaten liegen dementsprechend die Mikroorganismen in getrockneter Form vor und die sie umhüllende wasserlösliche oder wasserquellbare Schutzschicht ist ebenfalls in im wesentlichen wasserfreiem Zustand anwesend.

Die erfindungsgemässen flüssigen Präparate enthalten im allgemeinen $10^7$ bis $10^{10}$ von der Schutzschicht umhüllte Mikroorganismen pro ml Oel, und vorzugsweise $10^8$ bis $10^9$ von der Schutzschicht umhüllte Mikroorganismen pro ml Oel.

Das Material der Schutzschicht wird in solchen Mengen eingesetzt, dass im trockenen Zustand ein Gewichtsteil an Mikroorganismen mit 0,5 bis 10 Gew.-Teilen, vorzugsweise 2 bis 6 Gew.-Teilen, des trockenen Materiales aus der wasserlöslichen oder wasserquellbaren Schutzschicht umhüllt sind.

Die erfindungsgemässen lebende Mikroorganismen enthaltenden Präparate enthalten vorzugsweise als Mikroorganismen Bakterien oder Pilze, gegebenenfalls können jedoch die Mikroorganismen auch entsprechend umhüllte Hefen sein.

Gemäss einer bevorzugten Ausführungsart der Erfindung ist das lebende Mikroorganismen enthaltende Präparat ein in der Humanmedizin oder Veterinärmedizin einsetzbares Präparat, welches solche Mikroorganismenstämme enthält, die sich in der Mikroorganismenflora des Magen-Darmtraktes vorteilhaft auswirken. Diese Mikroorganismen können beispielsweise Milchsäure bildende Bakterien sein.

Es ist bekannt, dass es vorteilhaft ist, bei der Aufzucht von Jungtieren, insbesondere jungen Schweinen, die Darmflora der Tiere mit einem Bakterienstamm von Streptokokkus faecium anzureichern. Es ist ferner bekannt, dass durch Verabreichung dieses Bakterienstammes auch das Wachstum der Tiere gesteigert werden kann. Wenn man zu diesem Zwecke ein getrocknetes pulverförmiges Präparat verwendet, das den Streptokokkus faecium enthält und dieses mit Wasser anrührt und sodann dem Futter beisetzt, dann ergeben sich die weiter vorne ausführlich erläuterten Nachteile bezüglich der unerwünschten Geruchsentwicklung auf Oberflächen, die mit dem fraglichen Bakterium verunreinigt sind. Erfindungsgemässe flüssige Präparate, die einen Bakterienstamm von Streptokokkus faecium, vorzugsweise den Stamm Streptokokkus faecium Cernelle 68, der von der wasserlöslichen oder in Wasser quellenden Schutzschicht umhüllt ist, in dem Oel dispergiert enthalten, können ohne vorherige Verdünnung direkt in der gewünschten Dosis an die Tiere verabreicht werden. Vorzugsweise sind diese Präparate in einem entsprechenden Behälter abgepackt, der es erlaubt, den Ferkelchen eine gewünschte Einzeldosis durch Einträufeln oder Einspritzen in das Maul zu verabreichen.

Gemäss einer weiteren Ausführungsart der Erfindung ist das lebende Mikroorganismen enthaltende Präparat ein bei der Züchtung von Nutzpflanzen oder Zierpflanzen eingesetztes Präparat, welches solche Mikroorganismenstämme enthält, die krankheitserregend gegenüber tierischen und pflanzlichen Schädlingen, insbesondere Schadinsekten, sind.

Bei der Verwendung derartiger Präparate kann man das flüssige Oel enthaltende Präparat direkt auf die Pflanzen oder ihre Umgebung aufbringen. Im allgemeinen ist es jedoch vorteilhafter, das fragliche Präparat, zusammen mit einem entsprechenden Dispergiermittel, in Wasser einzubringen und darin zu dispergieren und die entsprechend verdünnte Lösung, beziehungsweise Dispersion, dann auf die Pflanzen und/oder ihre Umgebung, beispielsweise den Erdboden, aufzusprühen, beziehungsweise die entsprechende Flüssigkeit als Giessflüssigkeit einzusetzen.

Derartige Präparate sind ferner auch geeignet, um gelagerte landwirtschaftliche Produkte oder Lebensmittel gegen den Angriff von Schädlingen, insbesondere tierischen Schädlingen, wie Schadinsekten, zu schützen.

Die erfindungsgemässen lebende Mikroorganismen enthaltenden Präparate können ferner bei der industriellen Herstellung von Nahrungsmitteln angewandt werden, beispielsweise um Milchsäuregärungen oder alkoholische Gärungen durchzuführen.

Bekanntlich sind sehr viele spezielle Käsesorten nur in sehr begrenzten Regionen herstellbar, weil die Erzeugung des fraglichen Käses von der in der speziellen Region vorliegenden Mikroorganismenpopulation abhängig ist, welche die Umwandlung des Milchkoagulates, das durch Milchsäuregärung oder Labzugabe gewonnen wurde, in die spezielle Käsesorte, bewirkt. Für die Käseherstellung sind natürlich auch die klimatischen Bedingungen wichtig, insbesondere Temperatur und Luftfeuchtigkeit, die während der Umwandlung des Milchkoagulates in Käse vorherrschen. Die klimatischen Bedingungen einer bestimmten Region können durch Einhaltung entsprechender Temperaturen und Luftfeuchtigkeiten auch an anderen Orten, beispielsweise einer im Tiefland liegenden Käserei, simuliert werden. Doch war es bei vielen Käsesorten bisher nicht möglich, sie an fremden Orten herzustellen, weil die fragliche Mikroorganismenpopulation dort nicht vorhanden war. Erfindungsgemässe Präparate, in welchen die von der wasserlöslichen oder in Wasser quellenden Schutzschicht umhüllten, in der Oelphase suspendierten Mikroorganismen diejenige Mikroorganismenpopulation sind, die zur Herstellung einer bestimmten Käsesorte nötig ist, können somit verwendet werden, um die fragliche Käsesorte an einem fremden Ort zu erzeugen.

Ein weiterer Gegenstand der vorliegenden Erfindung ist ein Verfahren zur Herstellung des erfindungsgemässen lebende Mikroorganismen enthaltenden Präparates. Dieses Verfahren ist dadurch gekennzeichnet, dass man die Mikroorganismen aus dem Zuchtmedium abtrennt und sie mit einem wässrigen Medium vermischt, welches das in der Oelphase unlösliche, wasserlösliche oder in Wasser quellende Material der Schutzschicht gelöst oder dispergiert enthält, diese Mischung mindestens so weit trocknet, bis die getrockneten Mikroorganismen von der im wesentlichen wasserfreien festen Schutzschicht aus dem wasserlöslichen oder in Wasser quellenden Material umhüllt sind und die umhüllten Mikroorganismen in der Oelphase, die aus einem Mineralöl und/oder pflanzlichen Oel und/oder tierischen Oel, und/oder synthetischen flüssigen Fettsäureester aufgebaut ist, suspendiert.

Besonders vorteilhaft ist es, die Trocknung der Mischung aus den Mikroorganismen und dem wässrigen Medium, welches das Material der Schutzschicht gelöst oder dispergiert enthält, als Gefriertrocknung durchzuführen. Es zeigte sich jedoch, dass während des Trocknungsvorganges die sich ausbildende Schutzschicht aus dem festen wasserlöslichen oder in Wasser quellenden Material die Mikroorganismen ausreichend schützt, sodass gegebenenfalls die Trocknung auch als Sprühtrocknung vorgenommen werden kann. Bei der Herstellung der erfindungsgemässen Präparate wird vorzugsweise ein Gewichtsteil des Materiales der Schutzschicht in 5 bis 15 Gew.-Teilen Wasser, vorzugsweise 8 bis 12 Gew.-Teilen Wasser, gelöst und die aus dem Zuchtmedium abgetrennten Mikroorganismen werden in dieser Lösung dispergiert, vorzugsweise in einem Gewichtsverhältnis von 1 Gew.-Teil der feuchten, abgetrennten Mikroorganismen zu 1 bis 10 Gew.-Teilen, vorzugsweise 3 bis 6 Gew.-Teilen, dieser wässrigen Lösung.

Als Oelphase können in den erfindungsgemässen Präparaten verschiedene pflanzliche oder tierische Oele eingesetzt werden. Es zeigte sich dabei, dass solche tierischen Oele und pflanzlichen Oele, die geringere Anteile an Resten ungesättigter Fettsäuren enthalten, besser geeignet sind, als solche tierischen Oele und pflanzlichen Oele, die einen höheren Anteil an Resten ungesättigter Fettsäuren aufweisen. Es wurden viele Oele getestet, wie zum Beispiel Olivenöl, Erdnussöl, Sojaöl, Reiskeimöl, Maisöl, Sonnenblumenöl, Rapsöl, Jojobaöl und andere. Es zeigte sich dabei, dass Erdnussöl besonders gut geeignet ist und auch mit Sonnenblumenöl und Reiskeimöl waren gut geeignet.

Auch mit sogenannten synthetischen Oelen konnten gute Erfolge erzielt werden. Diese unterscheiden sich von den tierischen und pflanzlichen Oelen, die bekanntlich hauptsächlich aus Fettsäuretriglyceriden bestehen, dadurch, dass sie Fettsäureester einwertiger Alkohole darstellen oder Mischungen derselben enthalten. Als Beispiel für synthetische flüssige Fettsäureester seien genannt: Myristinsäure-isopropylester, Palmitinsäure-isopropylester, Oelsäure-äthylester, Oleyl-oleat und ähnliche Produkte. Auch das sogenannte Neutralöl mit der Markenbezeichnung "Miglyol" liefert gute Ergebnisse.

Die durchgeführten Versuche zeigten ferner, dass solche erfindungsgemässen Präparate, die als Oelphase ein Mineralöl enthalten, eine längere Lagerfähigkeit besitzen, als solche Präparate, die ein pflanzliches und/oder tierisches Oel enthalten. Die besten Ergebnisse wurden erzielt, wenn als Oelphase Paraffinöl verwendet wurde.

Es wurden Vergleichsversuche durchgeführt, die zeigten, dass trockene Mikroorganismen, die von keiner Schutzschicht umhüllt sind, und in einer bestimmten Oelphase dispergiert sind, bei einer Lagerung bei Zimmertemperatur eine wesentlich geringere Lagerfähigkeit besitzen, als die gleichen Mikroorganismenstämme, die von der wasserlöslichen oder in Wasser quellenden Schutzschicht umhüllt sind und im trockenen Zustand ebenfalls in dem gleichen Oel suspendiert wurden. In den erfindungsgemässen Präparaten waren nach einer dreijährigen Lagerung bei Zimmertemperatur nur 20 bis 40 % der Mikroorganismen abgestorben. Im Gegensatz dazu waren bei den Präparaten zu Vergleichszwecken, bei welchen die gleichen Mikroorganismen in unumhüllten Zustand in der gleichen Oelphase dispergiert waren, bereits nach einer Lagerungszeit von drei Monaten bei Zimmertemperatur 20 bis 40 % der lebenden Mikroorganismen abgestorben.

Die getrockneten, von der Schutzschicht aus dem wasserlöslichen oder in Wasser quellenden Material umhüllten Mikroorganismen, besitzen bei Lagerung bei Zimmertemperatur eine höhere Ueberlebensrate als entsprechende, ohne umhüllende Schutzschicht getrocknete Mikroorganismen. Versuche zu Vergleichszwecken zeigten jedoch, dass entsprechende, von der wasserlöslichen oder wasserquellenden Schutzschicht umhüllte Mikroorganismen, die bei Zimmertemperatur trocken aufbewahrt, jedoch nicht in einer Oelphase dispergiert waren, keine ausreichende Haltbarkeit besitzen. Nach einer Lagerungszeit von zwei bis vier Wochen bei Zimmertemperatur waren schon 20 bis 40 % der Mikroorganismen abgestorben.

Ueberraschenderweise zeigte es sich jedoch, dass lebende Mikroorganismen, die nur von der wasserlöslichen oder in Wasser quellenden Schutzschicht umhüllt sind, kurzzeitig hohe Temperaturen überleben, wenn sie dabei von einem Feststoff oder Gemisch von Feststoffen umgeben werden, das fest zusammengepresst wird und so bei der weiteren Lagerung des Produktes eine puffernde Wirkung gegen den Zutritt von Luftsauerstoff oder atmosphärischer Feuchtigkeit zu den von der Schutzschicht umhüllten Mikroorganismen ausübt.

Ein weiterer Gegenstand der vorliegenden Erfindung ist daher ein pelletiertes Produkt, das dadurch gekennzeichnet ist, dass es lebende Mikroorganismen enthält, die von einer wasserlöslichen oder in Wasser quellenden Schutzschicht, die in Mineralöl, pflanzlichem Oel, tierischen Oelen, synthetischen flüssigen Fettsäureestern, sowie Mischungen davon, unlöslich ist, umhüllt sind, wobei in dem pelletierten Produkt die Mikroorganismen in einem fest zusammengepressten Feststoff oder Gemisch auf Feststoffen, eingebettet sind und diese zusammengepressten Feststoffe den Zutritt von Sauerstoff oder atmosphärischer Feuchtigkeit zu den eingebetteten Mikroorganismen hemmen.

Derartige pelletierte Produkte, die lebende Mikroorganismen enthalten, besitzen im Vergleich zu pulverförmigen lebende Mikroorganismen enthaltenden Produkten ferner den Vorteil der leichteren Dosierbarkeit.

Ein bevorzugtes, lebende Mikroorganismen enthaltendes, pelletiertes Produkt ist ein entsprechendes pelletiertes diätetisches Nahrungsmittel oder ein pelletierter Futtermittelzusatz. In diesem Falle sind die zusammengepressten Feststoffe, in denen die umhüllten Mikroorganismen eingebettet sind, proteinhaltige Bestandteile, stärkehaltige Bestandteile, Mineralstoffe oder Mischungen aus zwei oder mehr derartigen Komponenten. Gegebenenfalls enthalten diese Produkte ausserdem noch Vitamine.

Des weiteren betrifft die vorliegende Erfindung ein Verfahren zur Herstellung eines lebende Mikroorganismen enthaltenden pelletierten Produktes. Dieses Verfahren ist dadurch gekennzeichnet, dass man die lebenden Mikroorganismen aus dem Zuchtmedium abtrennt und sie mit einem wässrigen Medium vermischt, welches das in Mineralöl, pflanzlichem Oel, tierischem Oel, synthetischen flüssigen Fettsäureestern und Mischungen davon unlösliche, jedoch wasserlösliche oder in Wasser quellende Material der Schutzschicht gelöst oder dispergiert enthält, diese Mischung mindestens so weit trocknet, dass die Mikroorganismen von der festen Schutzschicht aus dem wasserlöslichen oder in Wasser quellenden Material umhüllt werden und die umhüllten Mikroorganismen mit dem Feststoff oder dem Gemisch aus Feststoffen, vermischt und diese Mischung in Anwesenheit von Dampf bei einer Temperatur von 70 - 90° C unter Druck pelletiert.

Wenn dieses Verfahren zur Herstellung von Futtermittelpellets oder Pellets eines diätetischen Nahrungsmittels angewandt wird, dann ist es nicht nötig, dass entsprechende proteinhaltige Bestandteile oder stärkehaltige Bestandteile in vollständig trockenem Zustand zugefügt werden, sondern sie können ohne weiteres ziemlich feucht sein, sofern gewährleistet ist, dass sofort nach der Vermischung der umhüllten Mikroorganismen mit diesen Produkten der Pelletierungsvorgang vorgenommen wird. Bei der Pelletierung entweicht nämlich die überschüssige Feuchtigkeit noch ehe die die Mikroorganismen umhüllende Schutzschicht in irgendeinem wesentlichen Ausmass gelöst wird oder zu quellen beginnt.

Die Erfindung sei nun anhand von speziellen Beispielen näher erläutert, die jedoch den Schutzbereich der vorliegenden Erfindung in keiner Weise einschränken sollen.

Beispiel 1

Herstellung eines Präparates, welches als Mikroorganismus ein Bakterium enthält

Als Bakterienstamm wurde der Stamm Streptococcus faecium SF68 verwendet. Dieser wurde in üblicher Weise gezüchtet und durch Zentrifugieren aus dem Zuchtmedium abgetrennt. Nach dem Waschen wurde die abgetrennte Bakterienmasse mit einer Lösung von einem Teil Gummiarabikum in neun Teilen Wasser vermischt. Das Gewichtsverhältnis von abzentrifugierten Bakterien zu der Gummiarabikumlösung betrug etwa 1 : 4.

Der so erhaltene Brei wurde dann gefriergetrocknet. Es bildeten sich dabei kleine Kügelchen, welche die mit der Gummiarabikum-Schutzschicht überzogenen Bakterien enthielten.

Das Produkt wurde von groben Partikeln abgesiebt und in der Oelphase suspendiert.

Die Menge an der Oelphase wurde dabei so gewählt, dass jeweils 10 - 100 x $10^8$ Mikroorganismen pro ml Oel enthalten waren.

Beispiel 2

Testung der Lagerfähigkeit

Es wurde das nach dem Verfahren gemäss Beispiel 1 hergestellte erfindungsgemässe Präparat getestet und ferner auch ein Präparat zu Vergleichszwecken, bei dem der gleiche Mikroorganismenstamm nach der Abtrennung aus dem Zuchtmedium gewaschen und ohne Zugabe einer Lösung von Gummiarabikum direkt gefrier-getrocknet wurde. Dieses gefriergetrocknete Produkt wurde in der Oelphase in einer solchen Menge suspendiert, dass wieder 10 - 100 x $10^8$ unumhüllte Mikroorganismen pro ml Oel enthalten waren.

Sowohl beim erfindungsgemässen Präparat als auch bei dem Präparat zu Vergleichszwecken, wurde als Oelphase Paraffinöl verwendet.

Bei den erfindungsgemässen Präparaten und den Präparaten zu Vergleichszwecken wurde am Beginn des Tests die Anzahl der lebenden Keime pro ml Paraffinöl bestimmt und nach einer Lagerung der Proben bei Zimmertemperatur wurde wieder die Anzahl der lebenden Keime pro ml Paraffinöl getestet. Die bei diesen Tests erzielten Ergebnisse sind in der nachfolgenden Tabelle zusammengestellt.

| Präparat | Anzahl der lebenden Keime pro ml | | Testdauer in Tagen | % der während der Testdauer abgestorbenen Mikroorganismen |
| --- | --- | --- | --- | --- |
| | bei Testbeginn | bei Testende | | |
| erfindungs- gemäss | $89 \cdot 10^8$ | $59 \cdot 10^8$ | 603 | 34 |
| erfindungs- gemäss | $77 \cdot 10^8$ | $52 \cdot 10^8$ | 603 | 32 |
| Vergleich | $182 \cdot 10^7$ | $133 \cdot 10^7$ | 98 | 27 |
| Vergleich | $147 \cdot 10^7$ | $96 \cdot 10^7$ | 98 | 35 |

Man sieht aus den in der Tabelle angeführten Werten, dass in den Präparaten zu Vergleichszwecken, nach einer Testzeit von nur 98 Tagen ein Verlust an lebenden Keimen von 25 %, bzw. 35 % aufgetreten war. Die erfindungsgemässen Präparate, bei welchen die gleichen Mikroorganismen, jedoch umhüllt von der Schutzschicht aus Gummiarabikum, dispergiert in dem Paraffinöl getestet wurden, waren nach einer Lagerungszeit von nahezu zwei Jahren bei Zimmertemperatur, nämlich einer Lagerung von 603 Tagen, nur 34 %, bzw. 32 % der am Testbeginn vorhandenen lebenden Mikroorganismen abgestorben.

**Patentansprüche**

1. Lebende Mikroorganismen enthaltendes Präparat, bei dem die Mikroorganismen in einer flüssigen Oelphase suspendiert sind, dadurch gekennzeichnet, dass das Präparat ein bei Zimmertemperatur lagerfähiges Präparat ist, und dass die Mikroorganismen, die in der Oelphase suspendiert sind, in getrockneter Form vorliegen und von mindestens einer wasserlöslichen oder in Wasser quellenden, im wesentlichen wasserfreien Schutzschicht umhüllt sind, die in der Oelphase unlöslich ist, wobei die Oelphase aus einem Mineralöl und/oder pflanzlichen Oel und/oder tierischen Oel, und/oder synthetischen flüssigen Fettsäureester aufgebaut ist, und wobei im trockenen Zustand ein Gewichtsteil an Mikroorganismen von 0,5 bis 10 Gew.-Teilen des Materiales aus der wasserlöslichen oder in Wasser quellenden Schutzschicht umhüllt ist.

2. Lebende Mikroorganismen enthaltendes Präparat nach Anspruch 1, dadurch gekennzeichnet, dass die die Mikroorganismen umhüllende wasserlösliche oder wasserquellbare Schutzschicht aus einem Material besteht, das bei Zimmertemperatur fest ist und von den Mikroorganismen nicht abbaubar ist.

3. Lebende Mikroorganismen enthaltendes Präparat nach Anspruch 1 oder 2, dadurch gekennzeichnet, dass die die Mikroorganismen umhüllende Schutzschicht eine Schutzschicht aus einem wasserlöslichen Zellulosederivat, einem wasserlöslichen Stärkederivat, einem wasserlöslichen Pflanzengummi, einem wasserlöslichen Pektin oder einer Mischung aus zwei oder mehr derartigen wasserlöslichen Produkten ist.

4. Lebende Mikroorganismen enthaltendes Präparat nach einem der Ansprüche 1 bis 3, dadurch gekennzeichnet, dass im trockenen Zustand ein Gewichtsteil an Mikroorganismen von 2 bis 6 Gew.-Teilen, des Materiales aus der wasserlöslichen oder wasserquellbaren Schutzschicht umhüllt sind.

5. Lebende Mikroorganismen enthaltendes Präparat nach einem der Ansprüche 1 bis 4, dadurch gekennzeichnet, dass es als Mikroorganismen Bakterien oder Pilze enthält.

6. Lebende Mikroorganismen enthaltendes Präparat nach einem der Ansprüche 1 bis 5, dadurch gekennzeichnet, dass es ein in der Humanmedizin oder Veterinärmedizin einsetzbares Präparat ist, welches solche Mikroorganismenstämme enthält, die sich in der Mikroorganismenflora des Magen-Darmtraktes vorteilhaft auswirken, vorzugsweise Milchsäure bildende Bakterien, vorzugsweise Bakterienstämme von Streptokokkus faecium, insbesondere Streptokokkus faecium Cernelle 68.

7. Lebende Mikroorganismen enthaltendes Präparat nach einem der Ansprüche 1 bis 5, dadurch gekennzeichnet, dass es ein bei der Züchtung von Nutzpflanzen oder Zierpflanzen eingesetztes Präparat ist, welches solche Mikroorganismenstämme enthält, die krankheitserregend gegenüber tierischen und pflanzlichen Schädlingen, insbesondere Schadinsekten, sind.

8. Verfahren zur Herstellung des lebende Mikroorganismen enthaltenden Präparates gemäss Anspruch 1, dadurch gekennzeichnet, dass man die Mikroorganismen aus dem Zuchtmedium abtrennt und sie mit einem wässrigen Medium vermischt, welches das in der Oelphase unlösliche, wasserlösliche oder in Wasser quellende Material der Schutzschicht gelöst oder dispergiert enthält, diese Mischung mindestens so weit trocknet, bis die getrockneten Mikroorganismen von der im wesentlichen wasserfreien festen Schutzschicht aus dem wasserlöslichen oder in Wasser quellenden Material umhüllt sind und die umhüllten Mikroorganismen in der Oelphase, die aus einem Mineralöl und/oder pflanzlichen Oel und/oder tierischen Oel, und/oder synthetischen flüssigen Fettsäureester aufgebaut ist, suspendiert.

9. Verfahren nach Anspruch 8, dadurch gekennzeichnet, dass man die Trocknung als Gefriertrocknung oder Sprühtrocknung durchführt und anschliessend dann das trockene umhüllte Material in der Oelphase dispergiert.

10. Verfahren nach Anspruch 8 oder 9 dadurch gekennzeichnet, dass man ein wasserlösliches Zellulosederivat, ein wasserlösliches Stärkederivat, einen wasserlöslichen Pflanzengummi, beispielsweise Gummiarabikum, ein wasserlösliches Pektin oder eine Mischung aus zwei oder mehr derartigen wasserlöslichen Produkten in Wasser löst, vorzugsweise in einer Menge von einem Gewichtsteil dieses Materiales der Schutzschicht, in 5 bis 15 Gew.-Teilen Wasser, und die abgetrennten Mikroorganismen mit dieser wässrigen Lösung in einem Gewichtsverhältnis von 1 Gew.-Teil Mikroorganismen zu 1 bis 10 Teilen, vorzugsweise 3 bis 6 Teilen, der wässrigen Lösung vermischt.

11. Pelletiertes Produkt, dadurch gekennzeichnet, dass es lebende Mikroorganismen enthält, die von einer wasserlöslichen oder in Wasser quellenden Schutzschicht, die in Mineralöl, pflanzlichem Oel, tierischen Oelen, synthetischen flüssigen Fettsäureestern, sowie Mischungen davon, unlöslich und bei Zimmertemperatur fest ist, umhüllt sind, wobei die Schutzschicht entsprechende Cellulosederivate, Stärkederivate, Pflanzengummen, Pektine oder Mischungen aus zwei oder mehr derartigen Produkten enthält und die von der Schutzschicht umhüllten Mikroorganismen in einem fest zusammengepressten Feststoff oder ein Gemisch aus Feststoffen, eingebettet sind und diese Feststoffe als Puffer für den Zutritt von Luftsauerstoff oder atmosphärischer Feuchtigkeit zu den von der Schutzschicht umhüllten lebenden Mikroorganismen wirken.

12. Pelletiertes Produkt nach Anspruch 11, dadurch gekennzeichnet, dass es ein pelletiertes diätetisches Nahrungsmittel oder ein Futtermittelzusatz ist, in dem die zusammengepressten Feststoffe, in welchen die umhüllten Mikroorganismen eingebettet sind, proteinhaltige und/oder stärkehaltige Produkte sind, und wobei gegebenenfalls in dem pelletierten Produkt noch Mineralstoffe oder Vitamine vorhanden sind.

13. Verfahren zur Herstellung des pelletierten Produktes gemäss Anspruch 11, dadurch gekennzeichnet, dass man die lebenden Mikroorganismen aus dem Zuchtmedium abtrennt und sie mit einem wässrigen Medium vermischt, welches das Material der Schutzschicht gelöst oder dispergiert enthält, diese Mischung mindestens so weit trocknet, dass die Mikroorganismen von der festen Schutzschicht aus dem wasser-löslichen oder in Wasser quellenden Material umhüllt werden und die umhüllten Mikroorganismen mit dem Feststoff oder dem Gemisch aus Feststoffen vermischt und diese Mischung in Anwesenheit von Dampf bei einer Temperatur von 70 - 90° C unter Druck pelletiert.

**Claims**

1. Preparation containing living microorganisms, in which the microorganisms are suspended in a fluid oil phase, characterised in that the preparation is a preparation which can be stored at room temperature and in that the microorganisms, which are suspended in the oil phase, are present in dried form and are encased by at least one protective layer which is water-soluble or swells in water, which layer is substantially free from water and is insoluble in the oil phase, with the oil phase being built up out of a mineral oil and/or vegetable oil and/or animal oil and/or synthetic fluid fatty acid ester and with, in the dry state, one part by weight in terms of microorganisms being encased by 0.5 to 10 parts by weight of the material of the protective layer which is water-soluble or swells in water.

2. Preparation containing living microorganisms according to claim 1, characterised in that the protective layer which encases the microorganisms and which is water-soluble or capable of swelling in water, consists of a material which is solid at room temperature and is not capable of being broken down by the microorganisms.

3. Preparation containing living microorganisms according to claim 1 or 2, characterised in that the protective layer which encases the microorganisms is a protective layer consisting of a water-soluble cellulose derivate, a water-soluble starch derivate, a water-soluble vegetable gum, a water-soluble pectin or a mixture of two or more of such water-soluble products.

4. Preparation containing living microorganisms according to one of the claims 1 to 3, characterised in that in the dry state one part by weight in terms of microorganisms is encased by 2 to 6 parts by weight of the material of the protective layer which is water-soluble or capable of swelling in water.

5. Preparation containing living microorganisms according to one of the claims 1 to 4, characterised in that it contains, as microorganisms, bacteria or fungi.

6. Preparation containing living microorganisms according to one of the claims 1 to 5, characterised in that it is a preparation which can be used in human medicine or veterinary medicine and which contains such microorganism strains which have advantageous effects in the microorganism flora of the gastro-intestinal tract, preferably bacteria which form lactic acid, preferably bacterial strains of Streptococcus faecium, in particular Streptococcus faecium cernelle 68.

7. Preparation containing living microorganisms according to one of the claims 1 to 5, characterised in that it is a preparation which is used in growing useful plants or ornamental plants and contains such microorganism strains which are pathogenic in relation to animal and vegetable pests, in particular insect pests.

8. Method for preparing the preparation containing living microorganisms according to claim 1, characterised in that the microorganisms are separated from the growing medium and are mixed with an aqueous medium which contains the material of the protective layer in a dissolved or dispersed manner, which material is insoluble in the oil phase, is water-soluble or swells in water, this mixture is dried at least to such an extent until the dried microorganisms are encased by the substantially water-free, solid protective layer consisting of the material which is water-soluble or swells in water and the encased microorganisms are suspended in the oil phase which is built up out of a mineral oil and/or vegetable oil and/or animal oil and/or synthetic fluid fatty acid ester.

9. Method according to claim 8, characterised in that the drying is carried out as freeze-drying or spray-drying and subsequently the dry, encased material is then dispersed in the oil phase.

10. Method according to claim 8 or 9, characterised in that a water-soluble cellulose derivate, a water-soluble starch derivate, a water-soluble vegetable gum, for example, gum arabic, a water-soluble pectin or a mixture of two or more of such water-soluble products is dissolved in water, preferably in a quantity of one part by weight of this material of the protective layer in 5 to 15 parts by weight water, and the separated microorganisms are mixed with this aqueous solution in a weight ratio of 1 part by weight microorganisms to 1 to 10 parts, preferably 3 to 6 parts, of the aqueous solution.

11. Pelletized product, characterised in that it contains living microorganisms which are encased by a protective layer which is water-soluble or swells in water and which is insoluble in mineral oil, vegetable oil, animal oils, synthetic fluid fatty acid esters and also mixtures thereof and is solid at room temperature, with the protective layer containing corresponding cellulose derivates, starch derivates, vegetable gums, pectins or mixtures of two or more of such products, and the microorganisms encased by the protective layer being embedded in a firmly compressed solid or a mixture of solids, and these solids acting as buffers for the access of oxygen of the air or atmospheric moisture to the living microorganisms encased by the protective layer.

12. Pelletized product according to claim 11, characterised in that it is a pelletized dietary foodstuff or a feedstuff additive, in which the compressed solids, in which the encased microorganisms are embedded, are products which contain protein and/or starch and with, if applicable, minerals or vitamins additionally being present in the pelletized product.

13. Method for preparing the pelletized product according to claim 11, characterised in that the living microorganisms are separated from the growing medium and are mixed with an aqueous medium which contains the material of the protective layer in a dissolved or dispersed manner, this mixture is dried at least to such an extent that the microorganisms are encased by the solid protective layer consisting of the material which is water-soluble or swells in water and the encased microorganisms are mixed with the solid or the mixture of solids and this mixture is pelletized in the presence of steam under pressure at a temperature of 70 - 90°C.

**Revendications**

1. Préparation contenant des microorganismes vivants, dans laquelle les microorganismes sont en suspension dans une oléophase liquide, caractérisée en ce que la préparation est une préparation susceptible d'être conservée à la température ambiante et que les microorganismes qui sont en suspension dans l'oléophase, se présentent sous forme séchée et sont enrobés d'au moins une couche de protection, sensiblement exempte d'eau, soluble dans l'eau ou gonflant dans l'eau, mais insoluble dans l'oléophase, laquelle oléophase se compose d'une huile minérale et/ou d'une huile végétale et/ou d'une huile animale et/ou d'esters d'acides gras, liquides, synthétiques, où, à l'état fondu, une partie en poids de microorganismes est enrobée de 0,5 à 10 parties en poids de la matière constituant la couche de protection soluble dans l'eau ou gonflant dans l'eau.

2. Préparation contenant des microorganismes vivants selon la revendication 1, caractérisée en ce que la couche de protection soluble dans l'eau ou gonflable dans l'eau, enrobant les microorganismes, se compose d'une matière qui est solide à la température ambiante et qui n'est pas dégradable par des microorganismes.

3. Préparation contenant des microorganismes vivants selon la revendication 1 ou 2, caractérisée en ce que la couche de protection qui enrobe les microorganismes est une couche de protection constituée d'un dérivé de cellulose soluble dans l'eau, d'un dérivé d'amidon soluble dans l'eau, d'une gomme végétale soluble dans l'eau, de pectine soluble dans l'eau, ou d'un mélange de deux ou de plus de deux produits solubles dans l'eau de ce genre.

4. Préparation contenant des microorganismes vivants selon l'une quelconque des revendications 1 à 3, caractérisé en ce que, à l'état sec, une partie en poids de microorganismes est enrobée de 2 à 6 parties en poids de la matière constitutive de la couche de protection soluble dans l'eau ou gonflable dans l'eau.

5. Préparation contenant des microorganismes vivants selon l'une quelconque des revendications 1 à 4, caractérisée en ce qu'elle contient des bactéries ou des champignons à titre de microorganismes.

6. Préparation contenant des microorganismes vivants selon l'une quelconque des revendications 1 à 5, caractérisée en ce qu'elle est une préparation utilisable en médecine humaine ou en médecine vétérinaire, qui contient des souches de microorganismes du genre de celles qui exercent une activité avantageuse sur la flore des microorganismes des voies gastro-intestinales, de préférence des bactéries engendrant de l'acide lactique, de préférence des souches de bactéries de Streptococcus faecium, plus particulièrement Streptococcus faecium Cernelle 68.

7. Préparation contenant des microorganismes vivants selon l'une quelconque des revendications 1 à 5, caractérisée en ce qu'elle est une préparation utilisée pour l'élevage ou la culture de plantes utiles ou de plantes ornementales, qui contient des souches de microorganismes du genre de celles qui ont une activité destructrice des organismes nuisibles, animaux et végétaux, plus particulièrement les insectes nuisibles.

8. Procédé de réalisation de la préparation contenant des microorganismes vivants selon la revendication 1, caractérisé en ce que l'on sépare les microorganismes du milieu de culture et on les mélange à un milieu aqueux qui contient la matière insoluble dans l'huile, soluble dans l'eau ou gonflant dans l'eau, constitutive de la couche protectrice, en solution ou en dispersion, on sèche le mélange au moins jusque dans la mesure où les microorganismes séchés soient enrobés de la couche de protection solide, sensiblement exempte d'eau, constituée de la matière soluble dans l'eau ou gonflant dans l'eau et on met les microorganismes enrobés en suspension dans la phase huileuse qui se compose d'une huile minérale et/ou d'une huile végétale et/ou d'une huile animale et/ou d'esters d'acides gras, liquides, synthétiques.

9. Procédé suivant la revendication 8, caractérisé en ce que l'on entreprend le séchage sous forme de lyophilisation (cryodessiccation) ou sous forme de séchage par pulvérisation et on disperse ensuite la matière enrobée et séchée dans l'oléophase.

10. Procédé suivant la revendication 8, caractérisé en ce que l'on dissout un dérivé de cellulose soluble dans l'eau, un dérivé d'amidon soluble dans l'eau, une gomme végétale soluble dans l'eau, par exemple la gomme arabique, une pectine soluble dans l'eau, ou un mélange de deux ou de plus de deux produits solubles dans l'eau de ce genre, dans de l'eau, de préférence en une proportion d'une partie en poids de cette matière constitutive de la couche de protection, dans 5 à 10 parties en poids d'eau et on mélange les microorganismes séparés à cette solution aqueuse dans un rapport pondéral de 1 partie en poids de microorganismes pour 1 à 10 parties, de préférence 3 à 6 parties, de la solution aqueuse.

11. Produit granulé, caractérisé en ce qu'il contient des microorganismes vivants, qui sont enrobés d'une couche de protection soluble dans l'eau ou gonflant dans l'eau qui est insoluble dans une huile minérale, une huile végétale, des huiles animales, des esters d'acides gras, liquides, synthétiques, comme aussi des mélanges de ceux-ci et qui est solide à la température ambiante, où la couche de protection contient des dérivés de cellulose, des dérivés d'amidon, des gommes végétales, des pectines, ou des mélanges de deux ou plus de deux de ces produits, appropriés et les microorganismes enrobés de la couche protectrice sont noyés dans un solide ou dans un mélange de solides, fermement comprimé et ces solides servent de tampon ou de barrière à l'accès des microorganismes vivants enrobés de la couche protectrice par l'oxygène de l'air ou d'humidité atmosphérique.

12. Produit granulé selon la revendication 11, caractérisé en ce qu'il est un additif pour aliments ou un aliment diététique, granulé, dans lequel les solides comprimés dans lesquels les microorganismes enrobés sont noyés, sont des produits contenant des protéines et/ou de l'amidon et où des substances minérales ou des vitamines sont éventuellement présentes dans le produit granulé.

13. Procédé de fabrication du produit granulé selon la revendication 11, caractérisé en ce que l'on sépare les microorganismes vivants du milieu de culture et on les mélange à un milieu aqueux qui contient la matière de la couche protectrice en solution ou en dispersion, on sèche ce mélange jusque dans une mesure au moins telle que les microorganismes soient enrobés de la couche de protection solide constituée de la matière soluble dans l'eau ou gonflant dans l'eau et on mélange les microorganismes enrobés au solide ou au mélange de solides et on granule ce mélange à une température de 70-90°C, sous pression et en l'absence de vapeur d'eau.